(19) Europäisches Patentamt
European Patent Office
Office européen des brevets

(11) Veröffentlichungsnummer: **0 385 515 B1**

(12) **EUROPÄISCHE PATENTSCHRIFT**

(45) Veröffentlichungstag der Patentschrift: **13.04.94**

(51) Int. Cl.[5]: **C07D 333/68**, C07D 409/04

(21) Anmeldenummer: **90107116.7**

(22) Anmeldetag: **27.11.84**

(60) Veröffentlichungsnummer der früheren Anmeldung nach Art. 76 EPÜ: **0 146 797**

# (54) 2-Aminothiophenverbindungen.

(30) Priorität: **07.12.83 DE 3344294**

(43) Veröffentlichungstag der Anmeldung:
**05.09.90 Patentblatt 90/36**

(45) Bekanntmachung des Hinweises auf die Patenterteilung:
**13.04.94 Patentblatt 94/15**

(84) Benannte Vertragsstaaten:
**CH DE FR GB LI**

(56) Entgegenhaltungen:
**EP-A- 0 150 319**
**FR-A- 2 128 234**
**US-A- 3 989 505**

(73) Patentinhaber: **BAYER AG**

**D-51368 Leverkusen(DE)**

(72) Erfinder: **Fürstenwerth, Hauke, Dr.**
**Unterölbach 3a**
**D-5090 Leverkusen 3(DE)**

EP 0 385 515 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind 2-Aminothiophene der allgemeinen Formel

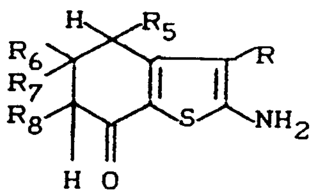

(I)

worin

R CN, $C_1$-$C_4$-Alkoxycarbonyl, $CONH_2$, CONH-$C_1$-$C_4$-Alkyl oder CON($C_1$-$C_4$-Alkyl)$_2$,

$R_5$, $R_6$, $R_7$ und $R_8$ H, $C_1$-$C_4$-Alkyl, Phenyl oder α-Furyl (die beiden letzteren jedoch insgesamt nur einmal)

bedeuten,

oder

$R_5$ und $R_6$ und/oder $R_7$ mit $R_8$ jeweils gemeinsam einen ankondensierten Benzolring bilden können.

Besonders bevorzugt sind Verbindungen der Formel I, worin R CN bedeutet.

Unter diesen sind wieder solche Typen der angegebenen Formeln bevorzugt,

worin

$R_5$ - $R_8$ = ein Wasserstoffatom bedeuten.

Die neuen Verbindungen sind nach verschiedenen Verfahren zugänglich.

Ein Verfahren zur Herstellung der Verbindungen der Formel I ist dadurch gekennzeichnet, daß man Verbindungen, die in einer ihrer tautomeren Formen der Formel

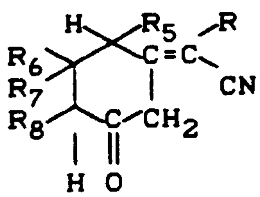

(II)

entsprechen mit elementarem Schwefel in einem unter den Reaktionsbedingungen indifferenten Medium ringschließend umsetzt.

Die Verbindungen der Formel II sind entweder bekannt oder nach an sich bekannten Methoden leicht herstellbar.

Auch die Reaktionsbedingungen der durch die Gegenwart des Schwefels gekennzeichneten Umsetzungen sind an sich bekannt.

In diesem Zusammenhang wird z.B. auf die folgende Literatur verwiesen.

Bücher: H.D. Hartough "Thiophene and Its Derivatives" und "Compounds with Condensed Thiohpene Rings", beide aus der Serie "The Chemistry of Heterocyclic Compounds", Interscience Publishers Inc., New York 1952.

Die Arbeiten von Gewald (et al) Z. Chem. 7 (1967), Heft 5, 186; Chem. Berichte 101, 1933; Chem. Berichte 98, (1965), 3571; Z. Chem. 2, (1962), 305. D.E. Wolf et al. "The Preparation of Thiophenes and Tetrahydrothiophenes", Organic Reactions 6, (1951), 410-468.

Als Lösungsmittel für die Reaktion eignen sich insbesondere Alkohole, Glykole, Glykolether oder Ether. Im einzelnen seien beispielsweise Methanol, Ethanol, Propanol, Glykol, Methyl-, Ethyl- oder Butylglykol sowie Tetrahydrofuran oder Dioxan genannt.

Einzelheiten der Reaktionsführung können den Beispielen entnommen werden, in denen sich Angaben über Teile und Prozente, sofern nicht anders vermerkt, auf das Gewicht beziehen.

Die neuen Verbindungen der Formel I sind wertvolle Zwischenprdukte zur Herstellung von Wirkstoffen auf dem Gebiet der Pharmazie und des Pflanzenschutzes, indem man diese Verbindungen beispielsweise mit Isocyanaten zu den entsprechenden Harnstoffen umsetzt.

Ganz besonders geeignet sind jedoch die neuen Aminothiophene als Diazokomponenten zum Aufbau von Azofarbstoffen, beispielsweise zur Herstellung von nebendichtearmen blaugrünen Azofarbstoffkörpern

für das fotografische Farbdiffusionsverfahren (vgl. z.B. US-PS 4 346 161 und EP-A-0150319 mit dem gleichen Prioritätsdatum wie die vorliegende Anmeldung).

Die Umsetzung der Verbindungen der Formeln V mit VI in Gegenwart von Schwefel, wird vorteilhaft unter Verwendung von nahezu stöchiometrischen Mengen der Reaktionsteilnehmer im organischen inerten Medium, gegebenenfalls in Gegenwart eines Katalysators durchgeführt.

Zweckmäßigerweise nimmt man die Umsetzung so vor, daß man die Verbindungen der Formel III sowie den Schwefel in feinverteilter Form in einem Lösungsmittel vorlegt und dann eine organische Base bei möglichst niedriger Temperatur, zutropft. Anschließend wird die Temperatur erhöht und vorzugsweise zwischen 30° und Siedetemperatur (Rückflußtemperatur) gehalten.

Nach beendeter Umsetzung kann dann das Reaktionsprodukt nach an sich üblichen Methoden isoliert werden, z.B. durch Fitrieren, gegebenenfalls Waschen und/oder Umkristallisieren.

Beispiel 1

16 Teile (Dicyanomethylen)-3-hydroxy-1-cyclohexan und 3,2 Teile Schwefel werden in 80 Teilen Ethanol vorgelegt. Nach Zugabe von 20 Teilen Triethylamin wird das Reaktionsgemisch 2 Stunden am Rückfluß gekocht. Man engt am Rotationsverdampfer zur Trockne ein, verrührt den Rückstand mit einer Mischung aus 100 Teilen Wasser und 30 Teilen Natriumhydrogensulfidlösung. Nach Isolieren, Waschen und Trocknen erhält man 10 Teile eines Produktes der Formel

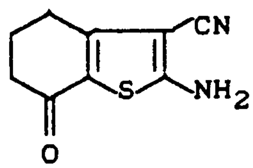

Fp.: 120° C

Beispiel 2

140 Teile Dimedon und 67 Teile Malodinitril werden mit 40 Teilen Ammoniumacetat in 800 Teilen Ethanol 1 Stunde am Rückfluß gekocht. Man kühlt ab auf 20°, gibt 32 Teile Schwefel und 150 Teile Triethylamin zu und erhitzt 3 Stunden am Rückfluß, destilliert 500 Teile Ethanol/Triethylamin-Gemisch ab, läßt kalt werden, isoliert, wäscht und trocknet. Es werden 80 g Produkt der Formel

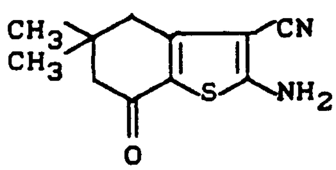

Fp.: 250° C

erhalten.

Arbeitet man nach den Angaben des Beispiel 51, setzt jedoch an Stelle von Dimedon die in folgender Tabelle aufgeführten Dicarbonylverbindungen ein, so erhält man entsprechende Thiophene.

| Bsp. Nr. | β-Diketon |
|---|---|
| 3 | 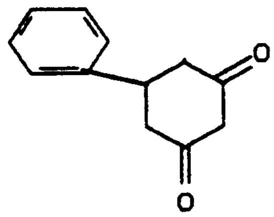 |
| 4 | 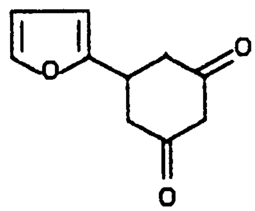 |
| 5 | 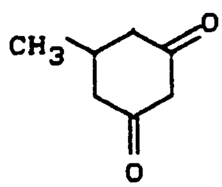 |

## Patentansprüche

**1.** Aminothiophene 1 der Formel

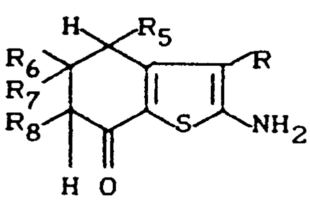

(I)

worin

| | |
|---|---|
| R | CN, $C_1$-$C_4$-Alkoxycarbonyl, $CONH_2$, CONH-$C_1$-$C_4$-Alkyl oder CON($C_1$-$C_4$-Alkyl)$_2$, |
| $R_5$, $R_6$, $R_7$ und $R_8$ | H, $C_1$-$C_4$-Alkyl, Phenyl oder $\alpha$-Furyl (die beiden letzteren jedoch insgesamt nur einmal) |

bedeuten,

oder

$R_5$ und $R_6$ und/oder $R_7$ mit $R_8$ jeweils gemeinsam einen ankondensierten Benzolring bilden können.

**2.** Aminothiophen der Formel

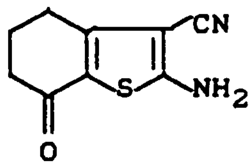 .

**3.** Verwendung der Aminothiohene gemäß Anspruch 1 zum Aufbau von Azofarbstoffen.

## Claims

**1.** Aminothiophenes 1 of the formula

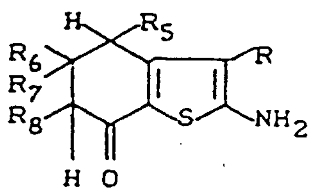 (I)

wherein

R denotes CN, $C_1$-$C_4$-alkoxycarbonyl, $CONH_2$, CONH-$C_1$-$C_4$-alkyl or CON($C_1$-$C_4$-alkyl) and

$R_5$, $R_6$, $R_7$ and $R_8$ denote H, $C_1$-$C_4$-alkyl, phenyl or $\alpha$-furyl (but the last two only once in total)

or

$R_5$ and $R_6$ and/or $R_7$ with $R_8$ in each case together can form a fused-on benzene ring.

**2.** Aminothiophene of the formula

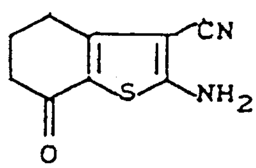 .

**3.** Use of the aminothiophenes according to Claim 1 for building up azo dyestuffs.

## Revendications

**1.** Aminothiophènes de formule

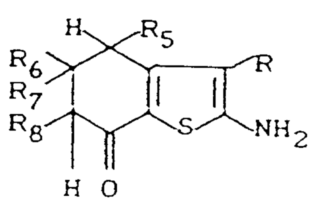 (I)

dans laquelle

R est un groupe CN, (alkoxy en $C_1$ à $C_4$)carbonyle, $CONH_2$, CONH-(alkyle en $C_1$ à $C_4$) ou CON(alkyle en $C_1$ à $C_4$),

$R_5$, $R_6$, $R_7$ et $R_8$ représentent H, un groupe alkyle en $C_1$ à $C_4$, phényle ou $\alpha$-furyle (les deux derniers n'apparaissant cependant qu'une seule fois au total)

ou bien

$R_5$ et $R_6$ et/ou $R_7$ et $R_8$ peuvent former dans chaque cas conjointement un noyau benzénique condensé.

**2.** Aminothiophène de formule

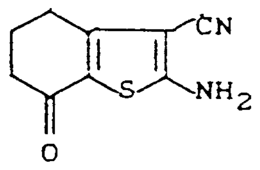 .

**3.** Utilisation des aminothiophènes suivant la revendication 1 pour la synthèse de colorants azoïques.